Europäisches Patentamt
European Patent Office
Office européen des brevets

(19)

(11) Publication number: **0 317 105 A2**

(12) # EUROPEAN PATENT APPLICATION

(21) Application number: 88310135.4

(51) Int. Cl.4: C07C 103/30 , A61K 47/00

(22) Date of filing: 28.10.88

(30) Priority: 20.11.87 JP 293176/87

(43) Date of publication of application:
24.05.89 Bulletin 89/21

(84) Designated Contracting States:
DE FR GB

(71) Applicant: AJINOMOTO CO., INC.
5-8, Kyobashi 1-chome, Chuo-ku
Tokyo 104(JP)

(72) Inventor: Fukushima, Koji
No. 2-23-5-3, Hijirigaoka
Tama-shi Tokyo(JP)
Inventor: Seto, Yoshiko
No. 1-2-3-406, Natsumidai
Funabashi-shi Chiba-ken(JP)
Inventor: Amino, Yusuke
No. 1-1, Suzuki-cho Kawasaki-ku
Kawasaki-shi Kanagawa-ken(JP)
Inventor: Nishikawa, Masahiko
No. 1-1, Suzuki-cho Kawasaki-ku
Kawasaki-shi Kanagawa-ken(JP)
Inventor: Toi, Koji
No. 1-1, Suzuki-cho Kawasaki-ku
Kawasaki-shi Kanagawa-ken(JP)

(74) Representative: Bond, Bentley George et al
HASELTINE LAKE & CO. Hazlitt House 28
Southampton Buildings Chancery Lane
London, WC2A 1AT(GB)

(54) **Phenylalanine derivative and its uses.**

(57) There is disclosed a phenylalanine derivative having the following structural formula:

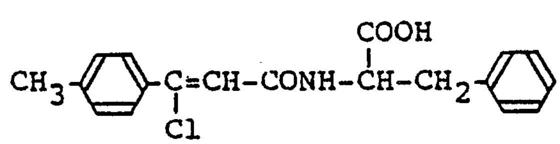

and salts thereof. The derivative and salt are useful for promoting the absorption of medicinal substances, in particular insulin.

EP 0 317 105 A2

# PHENYLALANINE DERIVATIVE AND ITS USES

This invention relates to a new phenylalanine derivative, N-(β-chloro-4-methylcinnamoyl)-phenylalanine, and non-toxic salts thereof, and to absorption accelerators containing the same as active ingredient. The object of the invention is to impart insulin and other drugs with the ability of being easily absorbed or to enhance this ability.

Phenylalanine derivatives (JP-A-190926/1984), benzoylpiperazine derivatives (JP-A-5115/1984) and hydroxybenzoate derivatives (Biochemica et Biophysica Acta, 775, 269-271, 1984) are already known as absorption accelerators. However, there has been a demand for a new absorption accelerator having more satisfactory properties for practical use.

Polypeptide drugs (e.g. insulin) are administered only by injection because if administered orally, they are deactivated by proteases contained in the digestive juices or cannot be absorbed through the intestinal tracts due to their high molecular weight. However, adminstration by injection gives physical pain and mental anguish to patients, particularly in the case of long-term treatment. Hence, there has been a great demand in recent years for the development of low toxicity absorption accelerators that can be applied by methods other than injection.

We have formerly invented absorption accelerators comprising a phenylalanine derivative as a main ingredient (JP-A-190926/1984 and JP-A-53954/1987). Much was expected from these agents for insulin therapy in the treatment of diabetes. However, agents of lower dosage and higher safety are still desired for insulin therapy requiring continuous administration over long periods.

Further studies to solve the above-mentioned problems have led us to accomplish the present invention.

Thus, the present invention provides the new phenylalanine derivative N-(β-chloro-4-methylcinnamoyl)-phenylalanine, having the following structural formula and salts thereof:

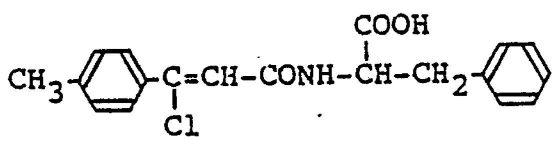

and new absorption accelerators comprising the same, in which the phenylalanine derivative or salt may be any of the L-, D- and DL-isomers.

The above mentioned N-(β-chloro-4-methylcinnamoyl)-phenylalanine and pharmaceutically acceptable salts thereof (inorganic salts such as sodium, potassium, calcium and aluminum salts, and organic salts such as the ammonium salt and salts with N-acetylgluosamine, arginine and lysine) can be administered orally or parenterally (for example, rectally) together with a medically active substance to make the latter more easily absorbed or to accelerate its absorption. In the case of insulin, for example, its oral and rectal absorption is significantly accelerated.

Of the phenylalanine derivatives which we proposed in the former invention, N-(β-fluoro-4-methylcinnmoyl)-L-phenylalanine may be mentioned as the most promising derivative for practical use.

Various tests revealed that the compound of the present invention is far better than the above compound in both its absorption accelerating effect and safety; that is, it exhibits high absorption accelerating effect with smaller doses and has a higher $LD_{50}$ value.

The absorption accelerator of the present invention is preferably used in an amount of 0.1 to 2000 mg, more preferably 0.2 to 500 mg, per 25 units of insulin.

This absorption accelerator may be used, together with a medically active substance, in the form of, for example, tablets, capsules, elixirs or suspensions.

A preferred unit dose of the N-(β-chloro-4-methylcinnamoyl)-phenylalanine or salt of the present invention is in the range of 0.1 to 1000 mg (e.g. a total of 0.2 to 2000 mg per day, subdivided in several doses). This amount may be varied within the above range depending on the severity of the disease, the body weight of patient, and other factors generally accepted by those skilled in the art.

The phenylalanine derivative of the present invention, a salt thereof, or a mixture thereof may be mixed with a drug and a physiologically acceptable additive such as a vehicle, carrier, excipient, binder, preservative, stabilizer and/or perfume, and fabricated into a commonly used dosage form so that each unit

dose will contain about 0.2 to 500 mg of the absorption accelerator and a prescribed amount of drug.

Additives that can be used for the preparation of tablets, capsules and other dosage forms are binders such as traganth, gum arabic, corn starch and gelatin; excipients such as crystalline cellulose; swelling agents such as corn starch, gelatinised starch and alginic acid; lubricants such as magnesium stearate; sweeteners such as sucrose, lactose and saccharin; and perfumes such as peppermint and cherry essence. In addition, capsules may also contain a liquid carrier such as a fatty oil. Similarly, tablets may also contain shellac, sugar or both as the coating material; and syrup and elixirs may also contain sucrose as a sweetener, methyl- or ethyl-paraben as a preservative, a perfume such as cherry or orange essence, and/or a colorant.

With insulin in particular, the preferred dosage form is an enteric preparation, which may be manufactured by usual methods, for example using an 8% aqueous solution of hydroxymethylcellulose as a primer and using a 10% aqueous solution of hydroxypropylmethylcellulose phthalate and 3% solution of polyacetin as a coating material.

The single Figure of the accompanying drawing shows the results of an activity test, as described in Example 4 below.

The invention will now be illustrated by the following Examples. As is apparent from the Examples, N-(β-chloro-4-methylcinnamoyl)-L-phenylalanine and N-(β-chloro-4-methylcinnamoyl)-D-phenylalanine of the present invention are better than conventional products in terms of safety and efficacy as an absorption accelerator for medically active substances, particularly insulin.

Example 1 Preparation of N-(β-chloro-4-methylcinnamoyl) = L-phenylalanine

To 6.0 g ( 30.5 mmol ) of β-chloro-4-methylcinnamic acid, prepared by the method described in J. Org. Chem., 40 3227 ( 1975 ), was added 40.0 ml thionyl chloride, the mixture was stirred at 90 °C for three hours, excess thionyl chloride was distilled off under reduced pressure, and the residue was dissolved in acetone.

L-phenylalanine ( 5.8 g, 30.5 mmol ) was dissolved together with 19.1 ml of 2N-NaOH solution ( 38.2 mmol ) in a mixture of 40.0 ml water and 80.0 ml acetone, this solution was cooled to below 10 °C, and the acetone solution of β-chloro-4-methylcinnamic acid chloride prepared above and 16.8 ml of 2N-NaOH solution ( 33.6 mmol ) were alternately added over a period of 20 minutes. Stirring was continued at room temperature for an additional 30 minutes, the reaction mixture was acidified by addition of 2N-HCl, and 200 ml water was then added slowly. The crystals which separated out were collected by filtration and dissolved in 200 ml ethyl acetate, and this solution was washed with 150 ml water and 100 ml saturated aqueous solution of sodium chloride. The organic layer was dried over anhydrous magnesium sulfate, the dried solution was concentrated, and the residue was recrystallized from ethyl acetate/n-hexane, giving 7.0 g of N-(β-chloro-4-methylcinnamoyl)-L-phenylalanine as white crystals melting at 168.5-169.5 °C.

Elemental analysis ( $C_{19}H_{18}NO_3Cl$ ):

| | C(%) | H(%) | N(%) | Cl(%) |
|---|---|---|---|---|
| Found | 66.41 | 5.20 | 4.02 | 10.48 |
| Calcd. | 66.37 | 5.28 | 4.08 | 10.31 |

Specific rotation: $[\alpha]_D^{24}$ , -14.2° ( c = 1.0, methanol )
Mass spectrum ( FD-MS ): $M^+$ = m/z 343
NMR spectrum ( $^1$H, 90 MHz ), δ( DMSO-$d_6$ ):
2.32 ( s, 3H ), 2.75-3.30 ( m, 2H ), 4.40-4.70 ( m, 1H ), 6.75 ( s, 1H ), 7.13 ( s, 5H ), 7.00-7.55 ( m, 4H ), 8.16 ( d, 1H ), 11.40 ( br. s, 1H )

Example 2 Preparation of N-(β-chloro-4-methylcinnamoyl)-D-phenylalanine

N-(β-chloro-4-methylcinnamoyl)-D-phenylalanine ( 6.50 g ) was obtained as white crystals melting at 168.5-169.5 °C in much the same manner as in Example 1 except that D-phenylalanine was used in place of L-phenylalanine.

Elemental analysis ( $C_{19}H_{18}NO_3Cl$ ):

| | C(%) | H(%) | N(%) | Cl(%) |
|---|---|---|---|---|
| Found | 66.58 | 5.47 | 4.09 | 10.36 |
| Calcd. | 66.37 | 5.28 | 4.08 | 10.31 |

Specific rotation: $[\alpha]_D^{25}$ , +14.3° ( c=1.0, methanol )
Mass spectrum ( FD-MS ): $M^+$ = m/z 343
NMR spectrum ( $^1H$, 90 MHz ), δ( DMSO-$d_6$ ):
2.32 ( s, 3H ), 2.75-3.30 ( m, 2H ), 4.40-4.70 ( m, 1H ), 6.75 ( s, 1H ), 7.00-7.55 ( m, 9H ), 8.16 ( d, 1H )

Example 3 ( Activity test using mice )

Each of the absorption accelerators prepared above was suspended in 0.5% CMC/0.05M Tris-HCl buffer ( pH 7.8 ), insulin was admixed to this suspension, and the resulting mixture was orally administered to a group of female ICR-CDI mice ( five head of 5- to 7-week age ) fasted for 18 hours ( insulin: 0.5 U/10 g, absorption accelerator: 3 mg/10 g ). The rate of reduction in blood glucose (%) and the factor of increase in the insuline level in the blood, in comparison with the data of the control group, were measured at definite intervals.

The result obtained is summarized in Table 1 below, in which 1) the upper figures in each column show the rate of reduction in blood glucose (%) and 2) the lower figures show the factor of increase in blood insulin level, while - indicates that no increase in blood insulin level was observed.

As can be seen from the table, N-(β-chloro-4-methylcinnamoyl)-L-phenylalanine and N-(β-chloro-4-methylcinnamoyl)-D-phenylalanine of this invention are far more effective than conventional drugs in reducing the level of glucose in the blood and increasing the amount of insulin in the test using mice.

Table 1

| Result of Activity Test Using Mice | | | |
|---|---|---|---|
| Sample | 1) Rate of reduction in blood glucose level 2) Factor of increase in blood-insulin level | | |
| | 15 minutes | 30 minutes | 60 minutes |
| N-(β-chloro-4-methylcinnamoyl)-L-phenylalanine | 1) 42.1% | 1) 21.6% | 1) 12.2% |
| | 2) 10.5 | 2) 2.0 | 2) 2.1 |
| N-(β-chloro-4-methylcinnamoyl)-D-phenylalanine | 1) 35.7% | 1) 47.8% | 1) 42.8% |
| | 2) 2.1 | 2) 3.3 | 2) - |

Example 4 ( Activity test using dogs )

An absorption acceleration test was conducted using dogs to compare the absorption accelerators of this invention with a known absorption accelerator, N-(α-fluoro-4-methylcinnamoyl)-L-phenylalanine.

Gelatin capsules containing 50 U/Kg insulin and 25 mg/Kg sodium salt of absorption accelerator were orally administered to male beagles of 6- to 8-month age fasted for 18 hours. In this test, the same two dogs were used for each of test samples.

A blood sample was taken from each dog at definite intervals from the peripheral vein in a paw, the amount of glucose in the serum was measured, and the average of two head was calculated. The result is sumarized in the figure.

Compared with N-(α-fluoro-4-methylcinnamoyl)-L-phenylalanine, N-(β-chloro-4-methylcinnamoyl)-L-phenylalanine and N-(β-chloro-4-methylcinnamoyl)-D-phenylalanine of this invention showed far higher effect of reducing the glucose level in the serum over longer periods.

Example 5 ( Toxicity test )

A known absorption accelerator, N-(α-fluoro-4-methylcinnamoyl)-L-phenylalanine, and the two absorption accelerators of this invention were each suspended in 0.5% solution of CMC sodium salt at different concentrations, each suspension was orally administered to male ICR mice of 4-week age ( each group consisting of 10 head ), and $LD_{50}$ values were measured based on the observations after two weeks. The result is summarized in Table 2, in which the figures show the number of living mice when 1.0 g/Kg and 3.0 g/Kg of the compounds were administered.

As is apparent from the table, N-(β-chloro-4-methylcinnamoyl)-L-phenylalanine and N-(β-chloro-4-methylcinnamoyl)-D-phenylalanine of this invention have $LD_{50}$ values equal to, or higher than, that of the known absorption accelerator.

Table 2

| Sample | Number of living mice* | |
|---|---|---|
| | 1.0 g/Kg | 3.0 g/Kg |
| N-(α-fluoro-4-methylcinnamoyl)-L-phenylalanine | 10 | 6 |
| N-(β-chloro-4-methylcinnamoyl)-L-phenylalanine | 10 | 10 |
| N-(β-chloro-4-methylcinnamoyl)-D-phenylalanine | 10 | 6 |

* Number of living mice among 10 head

**Claims**

1. A phenylalanine derivative having the following structural formula:

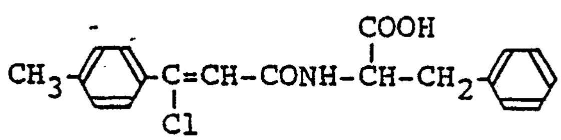

2. A salt of a phenylalanine derivative as claimed in claim 1.

3. An absorption accelerator comprising a phenylalanine derivative as claimed in claim 1 or a salt as claimed in claim 2.

4. A pharmaceutical composition comprising (a) a phenylalanine derivative as claimed in claim 1 or a salt as claimed in claim 2, and (b) a medicinal substance.

5. A composition as claimed in claim 4, wherein the medicinal substance is insulin.

6. A composition as claimed in claim 4 or 5, in the form of a tablet, a capsule, an elixir or a suspension.

Figure

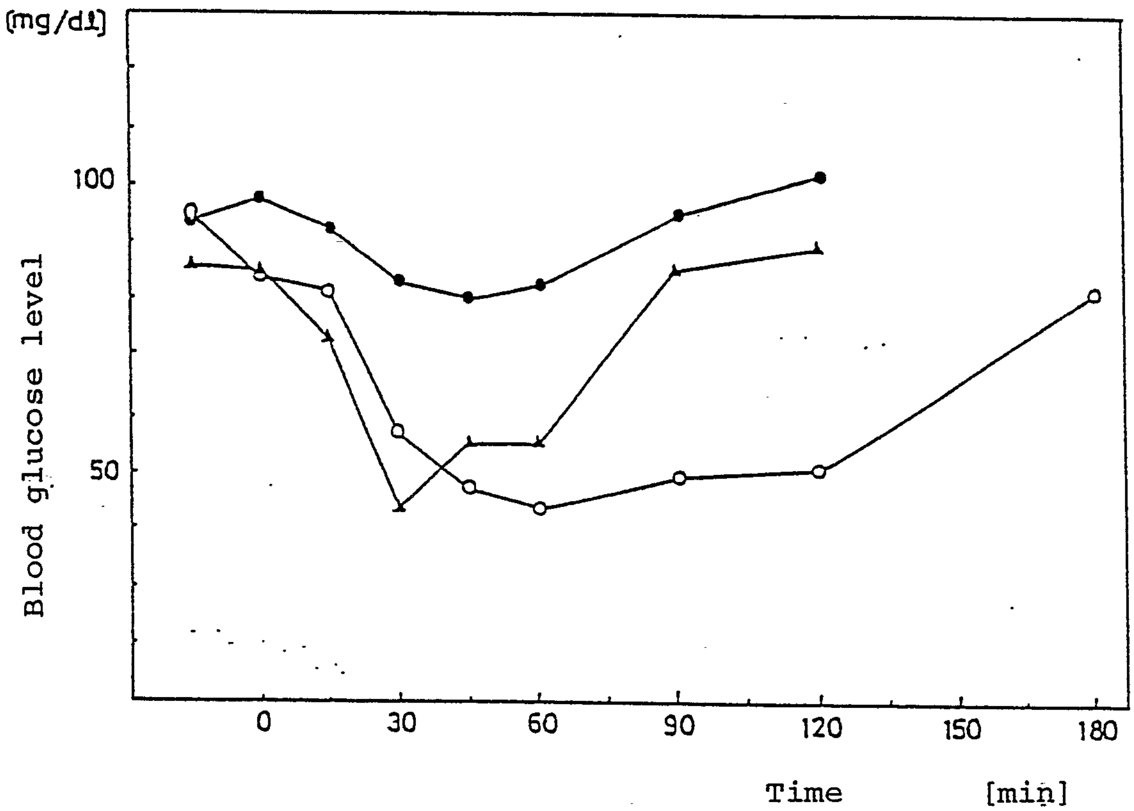


● N-(α-fluoro-4-methylcinnamoyl)-L-phenylalanine

○ N-(β-chloro-4-methylcinnamoyl)-L-phenylalanine

▲ N-(β-chloro-4-methylcinnamoyl)-D-phenylalanine